# EUROPEAN PATENT APPLICATION

(11) **EP 2 075 306 A2**
(43) Date of publication of application: **01.07.2009**
(21) Application number: 08021922.3
(22) Date of filing: 17.12.2008
(51) Int. Cl.: C09K 11/06, H05B 33/14

(54) **Organic electroluminescence material and element using the same**

(30) Priority: 25.12.2007 JP 2007332578; 10.11.2008 JP 2008287552
(71) Applicant: Yamagata Promotional Organization for Industrial Technology, Yamagata-shi, Yamagata 990-2473 (JP)
(72) Inventor: Oda, Atsushi, Yamagata-shi Yamagata 990-2743 (JP); Takayuki, Horiuichi, Yamagata-shi Yamagata 990-2743 (JP); Kimura, Masato, Yamagata-shi Yamagata 990-2743 (JP)
(74) Representative: HOFFMANN EITLE

(57) **Abstract**

An organic EL element having one or a plurality of organic layers including a light emitting layer between a pair of electrodes is arranged such that at least one layer of the above-mentioned organic layers contains a compound as expressed by the following general formula (1) independently or as a mixture. 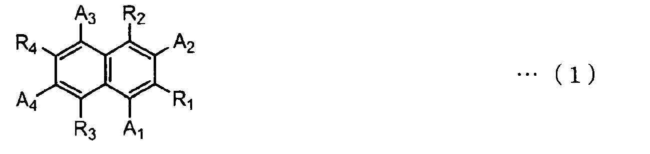 (where R₁-R₄ are selected from the group consisting of hydrogen, an alkyl group, a cycloalkyl group, an alkoxy group, a cycloalkoxy group, and an aryloxy group, and they may be the same groups or the groups different from one another, and A₁-A₄ are selected from the group consisting of a phenyl group which is either substituted or unsubstituted and a 5 or 6 member heterocyclic ring group which is either substituted or unsubstituted, and they may be the same groups or the groups different from one another.)

## Description

### BACKGROUND OF THE INVENTION

### Field of the Invention

The present invention relates to an organic electroluminescence material made of a novel naphthalene derivative for obtaining blue luminescence excellent in color purity, and to an organic electroluminescence element (hereinafter referred to as organic EL element) using the same.

### Description of the Related Art

Since the organic EL element is a self-luminescence type element which includes an organic compound as a light emitting material and allows luminescence at a high speed, it is suitable for displaying a video image, and it has features that allow an element structure to be simple and a display panel to be thin. Having such outstanding features, the organic EL element is spreading in everyday life as a cellular phone or a vehicle-mounted display.

Further, unlike LED, taking advantage of the features that it allows planar luminescence and is thin and lightweight, technical development as an illumination panel light source has also been carried out.

The above-mentioned organic EL element has been improved in luminescence efficiency by doping a host with a very small quantity of light emitting colorant. For this reason, the host material is an important material in order to obtain an efficient organic EL element.

In particular, in order to obtain a white light emitting element with high color rendering properties, the host material which can efficiently take blue luminescence from the blue-light emitting colorant is indispensable.

As the host materials for emitting blue light are conventionally known. For example, anthracene derivatives as shown in the following [Chemical Formula 1] and [Chemical Formula 2], a distyrylarylene derivative as shown in the following [Chemical Formula 3], a naphthalene derivative as shown in the following [Chemical Formula 4], etc. are known (for example, Japanese Patent Application Publication No. H11-312588, Japanese Patent Application Publication No. 2005-222948, Japanese Patent Application Publication No. H2-247278, and J. Shi, et al., Applied Physics Letters, 80 (17), p.3201).

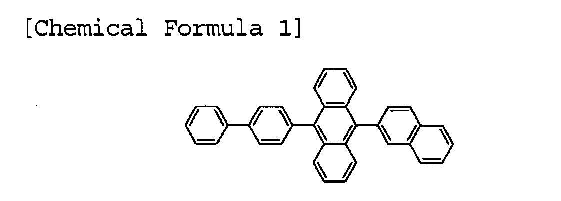

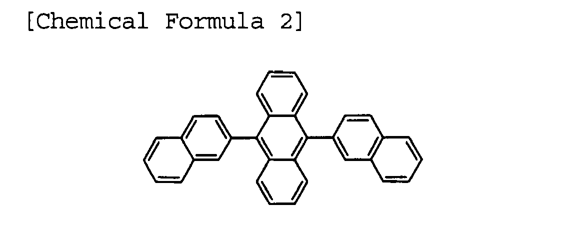

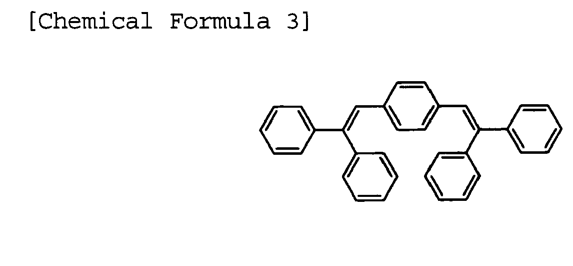

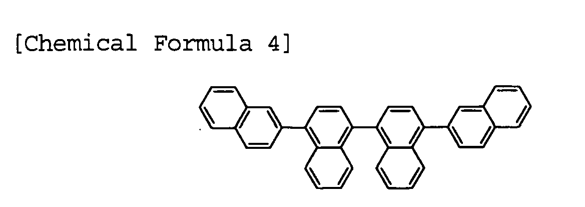

However, the materials as disclosed in the above-mentioned Japanese Patent Application Publication No. H11-312588, Japanese Patent Application Publication No. 2005-222948, Japanese Patent Application Publication No. H2-247278, and J. Shi, et al., Applied Physics Letters, 80 (17), p.3201 emit lights of bluish green and light blue. In the case where they are used as a host material, it is difficult to obtain blue luminescence excellent in color purity, which is required for the white light emitting element with high color rendering properties.

### SUMMARY OF THE INVENTION

The present invention arises in order to solve the above-mentioned technical problem, and aims at providing a new organic electroluminescence material which allows blue luminescent material excellent in color purity to emit light, and an organic EL element using the same.

The organic EL material in accordance with the present invention is expressed by the following general formula (1).

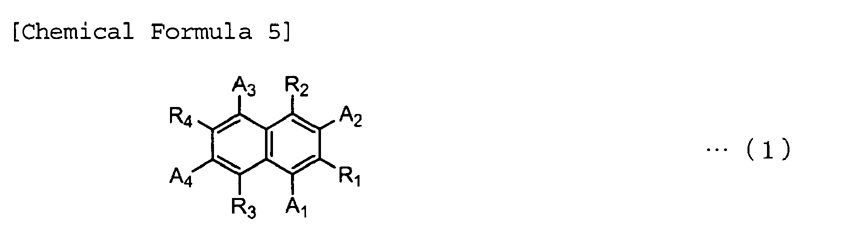

In the above-mentioned general formula (1), R₁-R₄ are selected from the group consisting of hydrogen, an alkyl group, a cycloalkyl group, an alkoxy group, a cycloalkoxy group, and an aryloxy group, and they may be the same groups or the groups different from one another. A₁-A₄ are selected from the group consisting of a phenyl group which is either substituted or unsubstituted and a 5 or 6 member heterocyclic ring group which is either substituted or unsubstituted, and they may be the same groups or the groups different from one another.

By using such a compound as the organic EL material, it is possible to obtain blue luminescence which is useful as a luminescence band and excellent in color purity.

Further, the organic EL element in accordance with the present invention is an organic EL element having one or a plurality of organic layers in which a light emitting layer is provided between a pair of electrodes, characterized in that at least one layer of the above-mentioned organic layers contains the above-mentioned organic EL material independently or as a mixture.

By using the above-mentioned organic EL material in accordance with the present invention, it is possible to construct an element which emits blue light excellent in color purity.

In the above-mentioned organic EL element, it is preferable that at least one layer of the above-mentioned organic layers is a light emitting layer containing the above-mentioned organic EL material as a host material and a fluorescence or phosphorescence emitting material as a guest material.

Further, as for the above-mentioned electrode, it is preferable that a transparent electroconductive thin-film is formed on a transparent substrate.

As described above, according to the organic EL material in accordance with the present invention, the blue luminescence excellent in color purity is obtained. Thus, by using this, it is possible to provide the white-light emitting element with high color rendering properties.

Therefore, it is expected that the organic EL element in accordance with the present invention would be applied to flat panel displays for OA computers and flat TV sets which nowadays require better color reproducibility, a light source for an illumination apparatus, a light source for a copying machine, light sources which take advantage of planar light-emitting members, such as backlight sources for a liquid crystal display, meters, etc., a display board, and a beacon light.

### BRIEF DESCRIPTION OF THE DRAWINGS

Fig. 1 is a graph showing a fluorescence spectrum of TMN1357.
Fig. 2 is a graph showing a fluorescence spectrum of N20.
Fig. 3 is a sectional view schematically showing a layer structure of an organic EL element with respect to Example 2.
Fig. 4 is a graph showing a light emission spectrum of the organic EL element with respect to Example 2.
Fig. 5 is a graph showing a light emission spectrum of the organic EL element with respect to Comparative Example 2.
Fig. 6 is a graph showing a light emission spectrum of the organic EL element with respect to Example 3.
Fig. 7 is a graph showing a light emission spectrum of the organic EL element with respect to Comparative Example 3.

### DESCRIPTION OF THE PREFERRED EMBODIMENTS

Hereafter, the present invention will be described in detail.

An organic EL material in accordance with the present invention is a compound expressed by the above-mentioned general formula (1).

Such a binaphthyl derivative is a novel compound which emits blue light excellent in color purity. By using this, it is possible to provide the white light emitting element with high color rendering properties.

In the above-mentioned general formula (1), R₁-R₄ are selected from the group consisting of hydrogen, an alkyl group, a cycloalkyl group, an alkoxy group, a cycloalkoxy group, and an aryloxy group, and they may be the same groups or the groups different from one another. Further, A₁-A₄ are selected from the group consisting of a phenyl group which is either substituted or unsubstituted and a 5 or 6 member heterocyclic ring group which is either substituted or unsubstituted, and they may be the same groups or the groups different from one another.

Among the above-mentioned substitution groups, the alkyl group indicates a saturated aliphatic hydrocarbon group, such as for example a methyl group, an ethyl group, a propyl group, a butyl group, etc., and it may be of a straight chain or may be of a branched chain.

The cycloalkyl group indicates a saturated alicyclic hydrocarbon group, such as for example, a cyclohexyl group, a norbornyl group, an adamantyl group, etc., and they may be either unsubstituted or substituted.

The alkoxy group indicates, for example, a saturated aliphatic hydrocarbon group through ether bonds, such as a methoxy group etc. , and it may be of a straight chain, or may be of a branched chain.

The cycloalkoxy group indicates a cyclic saturated aliphatic hydrocarbon group through ether bonds, such as for example, a cyclohexyl group etc., and it may be either unsubstituted or substituted.

The aryloxy group indicates an aromatic hydrocarbon group through ether bonds, such as for example a phenoxy group etc., and the aromatic hydrocarbon group may be either unsubstituted or substituted.

The substituted phenyl group indicates a phenyl group substituted with an alkyl group, a cycloalkyl group, an alkoxy group, a cycloalkoxy group, or an aryloxy group.

The heterocyclic group indicates a group which contains either nitrogen and oxygen or oxygen as a ring constituent element in addition to carbon. As examples thereof, there may be mentioned oxazole, oxadiazole, thiazole, thiadiazole, furan, furazan, thiophene, pyrane, thiopyrane, pyrrole, pyrazole, imidazoline, imidazole, pyrazine, pyridine, pyridazine, pyrimidine, triazole, triazine, etc., and they may be either unsubstituted or substituted.

Hereafter, among the compounds expressed by the above-mentioned general formula (1), particular examples of the substitution group combined with any of A₁-A₄ will be mentioned. In addition, in a phenyl group which is shown in the following [Chemical Formula 6] and is either substituted or unsubstituted and a 5 or 6 member heterocyclic ring group which is either substituted or unsubstituted, "X" indicates positions bonded with a naphthalene ring, i.e., bonding positions in A₁-A₄.

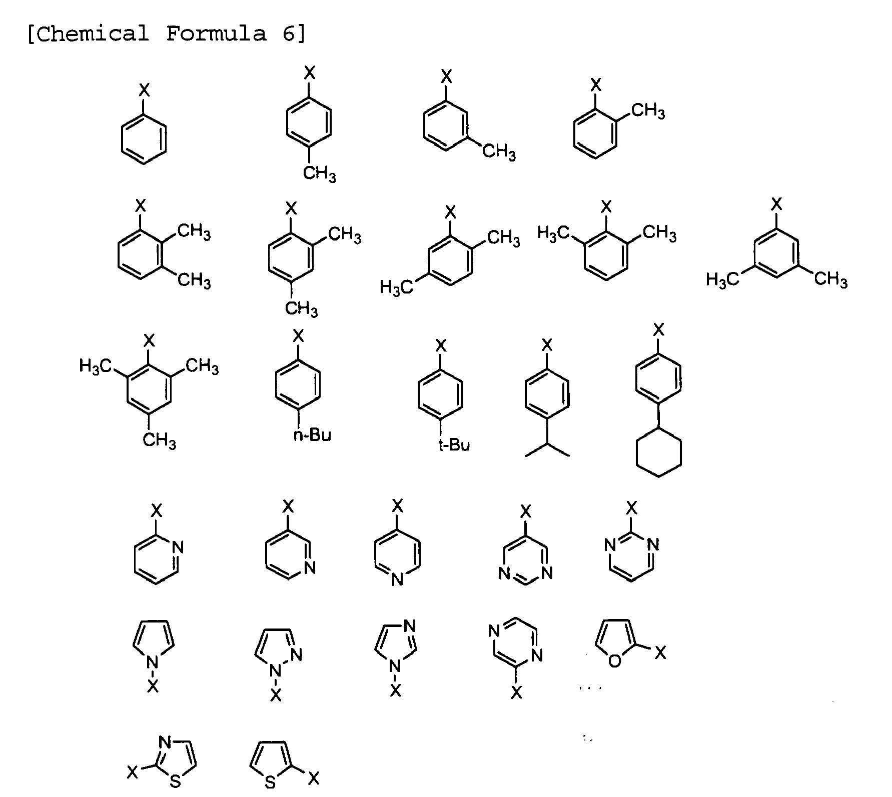

Furthermore, among the compounds expressed by the above-mentioned general formula (1), a structure of a particular compound is illustrated below.

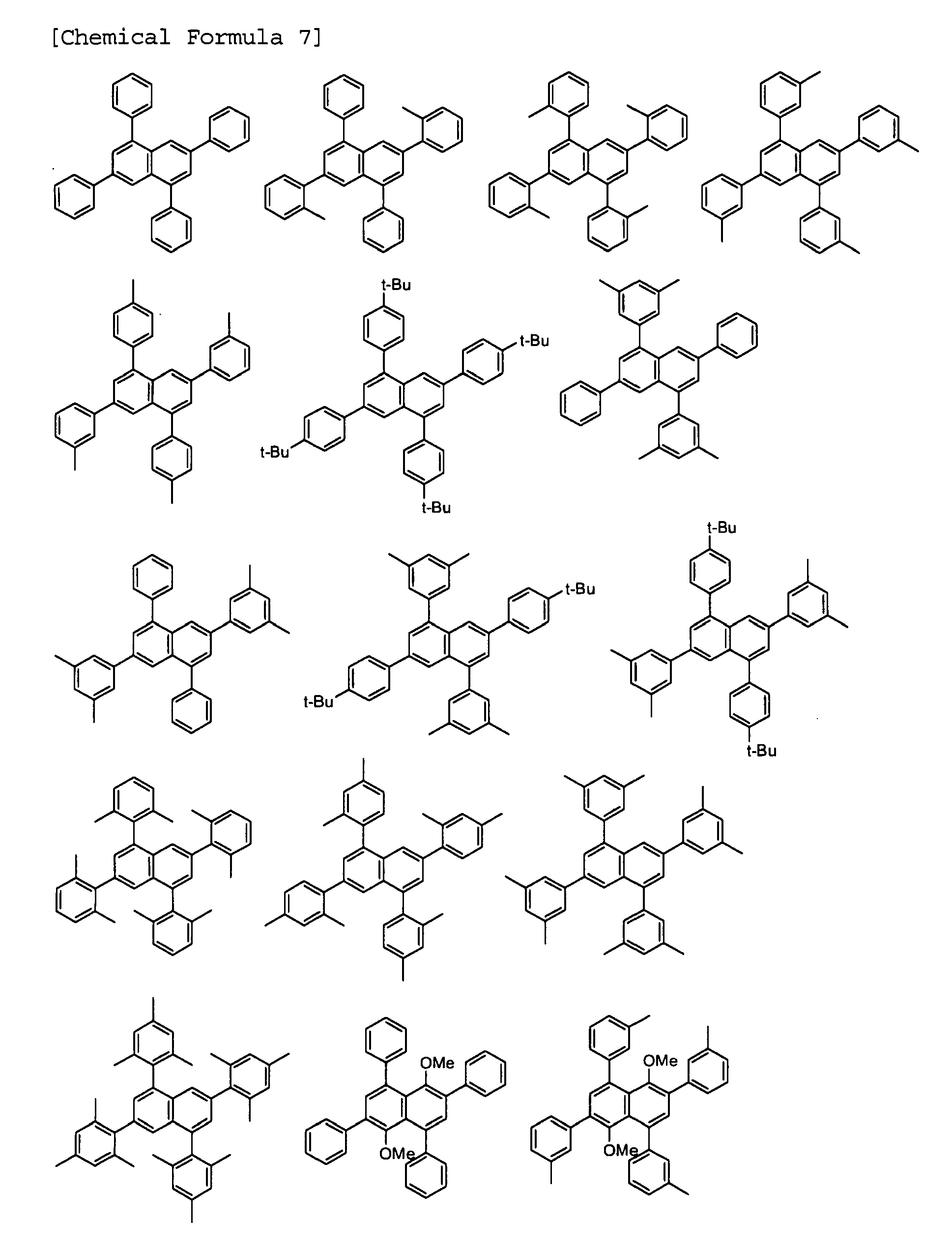

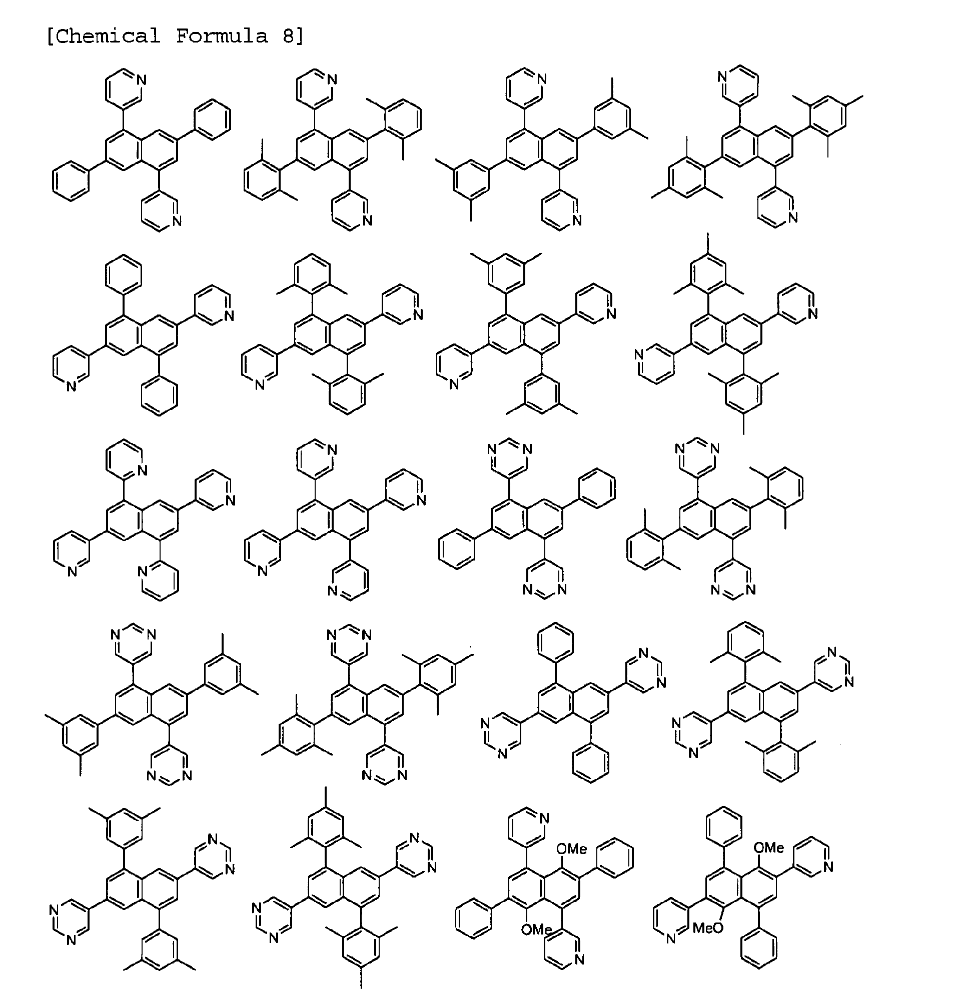

Among the compounds illustrated in the above-mentioned [Chemical Formula 7] and [Chemical Formula 8], there may be mentioned, as a representative, 1,3,5,7-tetra methylphenylnaphthalene (hereinafter abbreviated to TMN1357) expressed in the following [Chemical Formula 9] in particular.

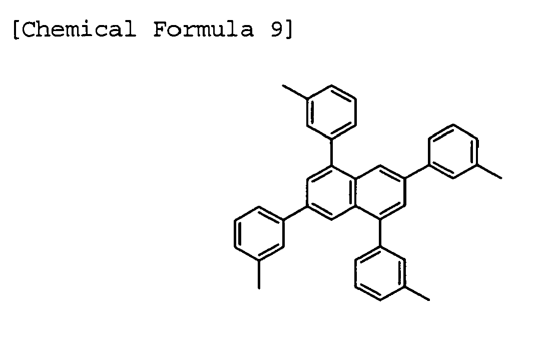

The compound expressed by the above-mentioned general formula (1) can be synthesized by way of a conventionally known synthetic reaction.

For example, diamino-substituted naphthalene is used as a material, and an amino group is substituted by a predetermined halogen group by way of the Sandmeyer reaction. As for the thus obtained di-halogen-substituted derivative, meta positions of the halogen substitution group are brominated in the presence of transition metal powder (preferably iron powder), to obtain a bromine derivative.

The naphthalene derivative expressed by the above-mentioned general formula (1) is synthesized by way of a coupling reaction between this bromine derivative and a corresponding cross coupling agent, using transition metal catalysts, such as Ni, Pd, etc.

At this time, by suitably selecting the halogen group for the Sandmeyer reaction, the asymmetrical compound expressed by the above-mentioned general formula (1) can also be synthesized in the coupling reaction using the transition metal catalysts, such as Ni, Pd, etc.

In addition, the material is not particularly limited, but a dihalogen derivative, a dihydroxy derivative, a dialkoxy derivative, etc. can also be used other than the above-mentioned diamino derivative of naphthalene.

Further, the compound expressed by the above-mentioned general formula (1) can also be synthesized by way of the Diels-Alder reaction.

The organic EL element in accordance with the present invention provided with the layer containing the above-mentioned organic EL material which can obtain blue luminescence excellent in color purity has a structure in which one or a plurality of organic layers are laminated between electrodes. As particular examples thereof, there may be mentioned a structure having "first electrode / light emitting layer / second electrode", "first electrode / hole transport layer / light emitting layer / electron transport layer / second electrode", "first electrode / hole transport layer / light emitting layer / second electrode", "first electrode / light emitting layer / electron transport layer / second electrode", etc.

Furthermore, it is also possible to employ the known lamination structure further including a hole injection layer, a hole transport light emitting layer, an electron injection layer, an electron transport light emitting layer, etc.

As for the electrode of the organic EL element in accordance with the present invention, it is preferable that the transparent electroconductive thin-film is formed on the transparent substrate.

The above-mentioned substrate is a support member of the organic EL element. In the case where the substrate side is a light emitting side, it is preferable to use a transparent substrate which is transmissive in visible light. It is preferable that the optical transmissivity is 80% or more. More preferably, it is 85% or more. Most preferably, it is 90% or more.

In general, the above-mentioned transparent substrate employs glass substrates made of, such as for example, optical glass (BK7, BaK1, F2, etc.), silica glass, non alkali glass, borosilicate glass, aluminosilicate glass, polymer substrate made of, such as for example, acrylic resins (PMMA, etc.), polycarbonate, polyether sulphonate, polystyrene, polyolefin, an epoxy resin, and polyethylene terephthalate, polyester, etc.

Although the above-mentioned substrate having a thickness of approximately 0.1-10 mm is usually used, it is preferable that the thickness is 0.3-5 mm in view of mechanical strength, weight, etc. More preferably it is 0.5-2 mm.

Further, in the present invention, it is preferable that the first electrode is provided on the above-mentioned substrate. Although this first electrode is usually an anode and is made of a metal, an alloy, an electroconductive compound, etc., with a large work function (4 eV or more), it is preferable to be formed as a transparent electrode on the above-mentioned transparent substrate.

In general, metal oxides, such as indium tin oxide (ITO), indium zinc oxide, zinc oxide, etc. are used for this transparent electrode. In particular, ITO is preferably used in terms of its transparency, conductivity, etc.

In order to secure the transparency and conductivity, it is preferable that a film thickness of this transparent electrode is 80-400 nm. More preferably, it is 100-200 nm.

It is preferable that the anode is usually formed by way of a sputtering method, a vacuum deposition method, etc., and formed as the transparent electroconductive thin-film.

On the other hand, in the case where the above-mentioned anode is the first electrode, a cathode of the second electrode which faces this anode is made of a metal, an alloy, and a conductive compound having a small work function (4eV or less). As examples thereof, there may be mentioned aluminum, an aluminum-lithium alloy, a magnesium-silver alloy, etc.

It is preferable that the film thickness of the above-mentioned cathode is 10-500 nm. More preferably, it is 50-200 nm.

The above-mentioned anode and cathode can be formed by forming a film by way of methods usually used, such as a sputtering method, an ion plating method, a vapor-depositing method, etc.

Respective materials used for the above-mentioned hole injection layer, hole transport layer, and hole transport light emitting layer are not particularly limited, but can be suitably selected from known materials to use.

In particular, they may be aryleneamines, such as bis(di(p-trill)aminophenyl)-1,1-cyclohexane (common name: TAPc), Spiro-TPD [Chemical Formula 11], etc., phenylenediamines, such as N,N'-diphenyl- N,N'-bis(3-methylphenyl)-1,1'-biphenyl-4,4'-diamine (common name: TPD), N,N'-diphenyl-N,N'-bis(1-naphthyl)-1,1'-biphenyl-4,4'-diamine (common name: alpha-NPD), etc., aryleneamines [Chemical Formula 11], such as TPTE etc., and arylamine derivatives, such as starburst amines [Chemical Formula 12], styrylamines [Chemical Formula 13], etc.

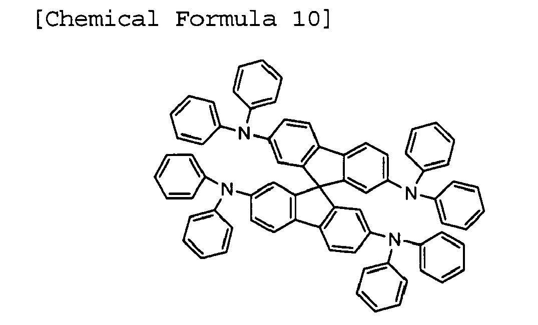

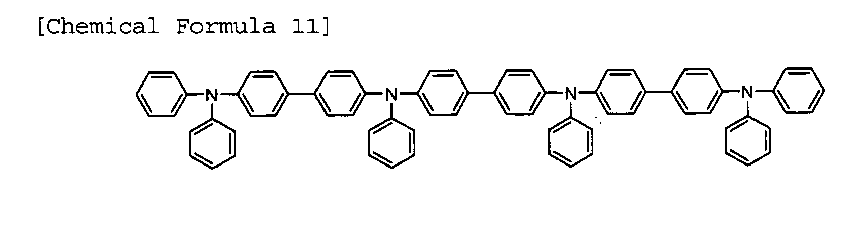

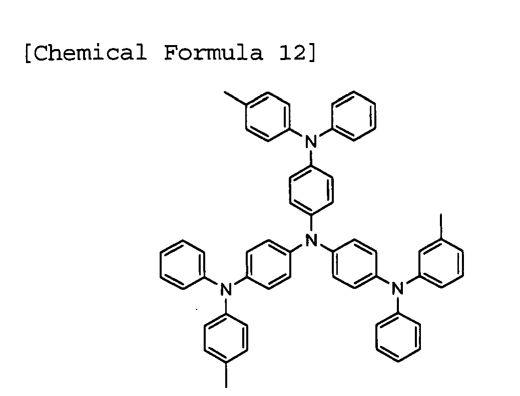

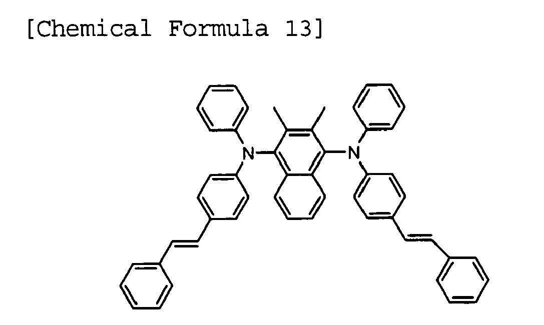

Further, it is also possible to use carbazole derivatives, such as bis(N-arylcarbazole) [Chemical Formula 14], bis(N-alkenylcarbazole), bis(N-alkylcarbazole), etc., a pyrazoline derivative, and styryl compounds and their derivatives, such as [Chemical Formula 15], [Chemical Formula 16], [Chemical Formula 17], etc.

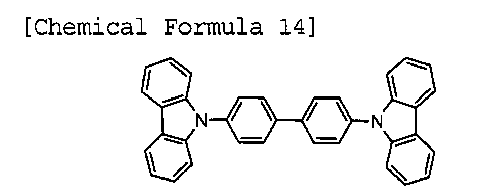

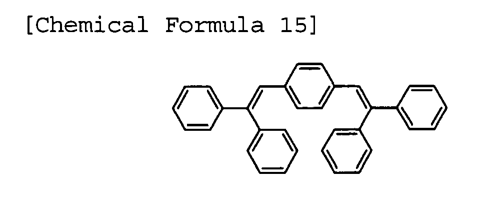

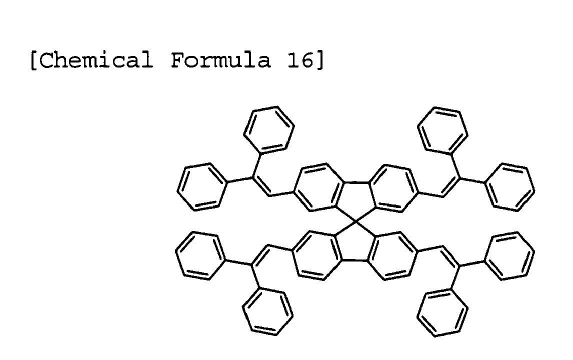

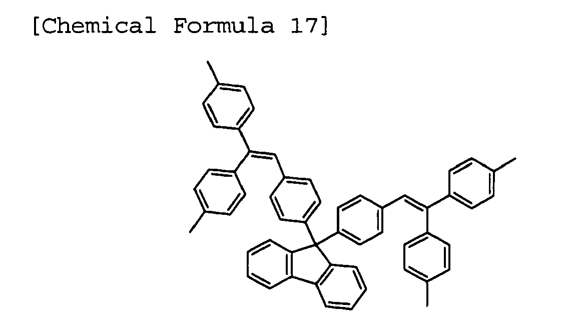

Alternatively, it is also possible to use fused multi-ring aromatic hydrocarbon compounds and those derivatives, such as anthracene, triphenylene, perylene, naphthalene, pyrene, coronene, chrysene, naphthacene, tetracene, phenanthrene, etc. as well as multi-ring compounds and those derivatives, such as paraterphenyl, quaterphenyl, m-phenylenes [Chemical Formula 18], [Chemical Formula 19], etc.

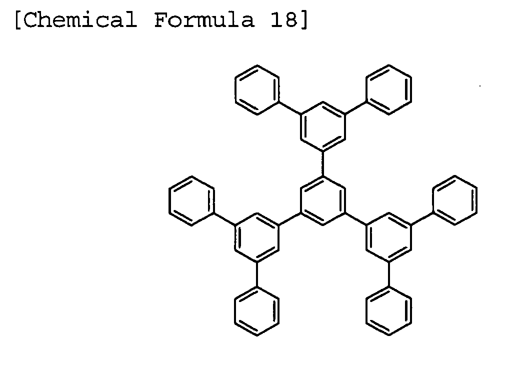

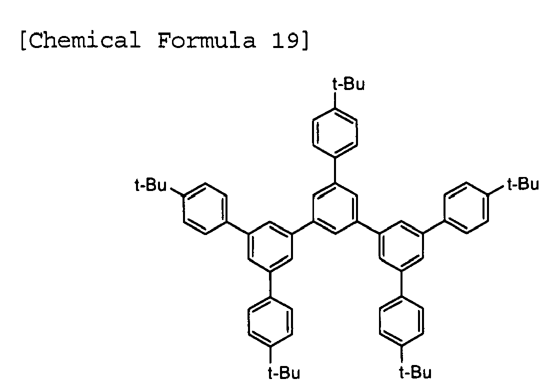

Furthermore, the hole injection layer, the hole transport layer, and the hole transport light emitting layer may employ the above-mentioned organic compound which is dispersed in a polymer, an oligomer, or a dendrimer, or which is polymerized, oligomerized or dendrimerized.

Still further, it is also possible to use so-called π-conjugated polymers etc., such as polyparaphenylenevinylene, polyfluorene, their derivatives, etc., the hole transport disconjugate polymer represented by poly(N-vinylcarbazole), σ-conjugated polymers represented by polysilanes, etc. Furthermore, so-called conjugated oligomers, such as a fluorene oligomer and its derivative, etc. can also be used.

Further, besides the above-mentioned materials, the hole injection layer may employ conductive polymers, such as metal phthalocyanines, non-metal phthalocyanines, carbon film, fluorocarbon film, polystyrene sulfonic acid (PEDOT-PSS) , polyaniline, etc.

Furthermore, the above-mentioned organic compound may be reacted with organic oxidizing dopants, such as tetracyanoquinodimethane, trinitrofluorenone, etc., and inorganic oxidizing dopants, such as vanadium oxide, molybdenum oxide, tungstic oxide, aluminum oxide, etc., to form a radical cation, and can be used as the hole injection transport layer. Although the oxidizing dopant concentration in this hole injection transport layer is not particularly limited, but it is preferable to be approximately 0.1 - 99 % by weight.

Yet further, respective materials used for the electron injection layer, the electron transport layer, and the electron transport light emitting layer are not particularly limited either, but can be suitably selected from the known materials to use.

As particular examples, there may be mentioned multi-ring compounds and those derivatives, such as paraterphenyl, quaterphenyl, m-phenylenes [Chemical Formula 18], [Chemical Formula 19], etc., and styryl compounds and those derivatives, such as [Chemical Formula 15], [Chemical Formula 16], [Chemical Formula 17], etc.

Further, it is also possible to use fused multi-ring aromatic hydrocarbon compounds and those derivatives, such as anthracene, triphenylene, perylene, naphthalene, pyrene, coronene, chrysene, naphthacene, tetracene, phenanthrene, etc., as well as heterocyclic compounds and those derivatives, such as phenanthroline, bathophenanthroline, bathocuproin, phenanthridine, acridine, quinoline, quinoxaline, pyridine [Chemical Formula 20], pyrimidine, pyrrole, pyrazole, pyridazine, pyrazine, phthalazine, naphthylidine, quinazoline, cinnoline, thiazole, oxadiazole, oxazole, triazine, phenazine, imidazole, benzoxazole, benzothiazole, benzoimidazole, triazole, porphyrin, etc.

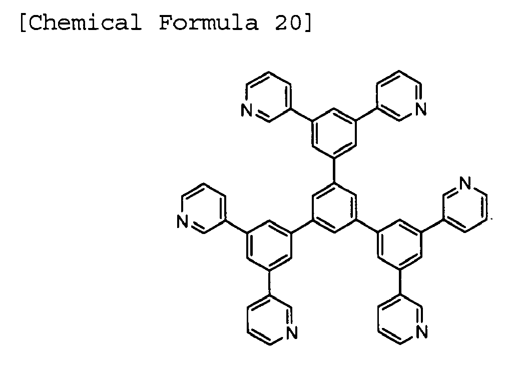

Further, it is also possible to use metal chelate complex materials, for example, a quinolinol aluminum complex, a benzoxazole zinc complex, a benzothiazole zinc complex, an azomethine zinc complex, a europium complex, an iridium complex, a platinum complex, etc., in which Al, Zn, Be, Ir, Pt, Tb, Eu, etc. are provided as central metals, and oxadiazole, thiadiazole, phenylpyridine, and quinoline structures are provided as ligands.

It is also possible to use organosilicon compounds and those derivatives, such as silole, siloxane, etc., organic boron compounds and those derivatives, such as triarylborane etc., and pentavalent phosphorus compounds and those derivatives etc., such as triarylphosphoxide etc.

Furthermore, the electron injection layer, the electron transport layer, and the electron transport light emitting layer may employ the above-mentioned organic compound which is dispersed in a polymer, an oligomer, or a dendrimer, or which is polymerized, oligomerized or dendrimerized.

Further, it is also possible to use so-called π-conjugated polymers, such as polyparaphenylenevinylene, polyfluorene, their derivatives, etc., and the electron transport disconjugate polymer etc. represented by polyvinyl oxadiazole. Furthermore, the so-called conjugated oligomers etc, such as a fluorene oligomer and its derivative, etc. can be used.

Besides the above-mentioned organic compounds, as the constituent material of the electron injection layer, it is possible to use single metals, such as Ba, Ca, Li, Cs, Mg, Sr, W, etc., metal fluorides, such as magnesium fluoride, calcium fluoride, strontium fluoride, barium fluoride, lithium fluoride, cesium fluoride, etc., metal alloys, such as an aluminum lithium alloy etc., metal oxides, such as magnesium oxide, strontium oxide, aluminum oxide, etc., and organometallic complexes, such as sodium polymethylmethacrylate polystyrene sulphonate etc.

Furthermore, the above-mentioned organic compound may be reacted with organic reducing dopants, such as 8-hydroxyquinoline Cs, Li organometallic complex, etc., to form a radical anion, which can be used as the electron injection transport layer.

Further, single metals, such as Ba, Ca, Li, Cs, Mg, Sr, W, etc., metal oxides, such as magnesium oxide, strontium oxide, aluminum oxide, etc., metal salts, such as magnesium fluoride, calcium fluoride, strontium fluoride, barium fluoride, lithium fluoride, cesium fluoride, cesium chloride, strontium chloride, etc., and inorganic reducing dopants may be mixed or dispersed to form a radical anion, which can be used as the electron injection transport layer.

Although the reducing dopant concentration in the above-mentioned electron injection transport layers is not particularly limited, it is preferable to be approximately 0.1 - 99 % by weight.

Further, it is possible to constitute the organic layer of the organic EL element in accordance with the present invention by using a bipolar material. By bipolar material is meant a material which can convey both the hole and the electron and may emit light by itself.

Respective materials used for the bipolar transport layer and a bipolar light emitting layer are not particularly limited.

As examples thereof, there may be mentioned styryl compounds and those derivatives, such as [Chemical Formula 15], [Chemical Formula 16], [Chemical Formula 17], etc., multi-ring aromatic compounds and those derivatives, such as paraterphenyl, quaterphenyl, m-phenylenes [Chemical Formula 18], [Chemical Formula 19] etc., fused multi-ring aromatic hydrocarbon compounds and those derivatives, such as anthracene, triphenylene, perylene, naphthalene, pyrene, coronene, chrysene, naphthacene, tetracene, phenanthrene, etc., carbazole derivatives [Chemical Formula 14], such as bis(N-arylcarbazole), bis(N-alkenylcarbazole), and bis(N-alkylcarbazole), heterocyclic compounds, such as thiophene etc.

Further, as examples other than these derivatives etc., there may be mentioned 4,4-bis(2,2-diphenyl-ethene-1-yl)diphenyl (DPVBi) [Chemical Formula 21], spiro6 [Chemical Formula 22], 2,2',7,7'-tetrakis(carbazole-9-yl)-9,9'-spiro-bifluorene [Chemical Formula 23], 4,4'-di(N-carbazolyl)-2',3',5',6'-tetraphenyl-p-terphenyl [Chemical Formula 24], 1,3-bis(carbazole)-9-yl-benzene [Chemical Formula 25], and 3-tert-butyl-9,10-di(naphtha-2-yl)anthracene (common name: TBADN) [Chemical Formula 26].

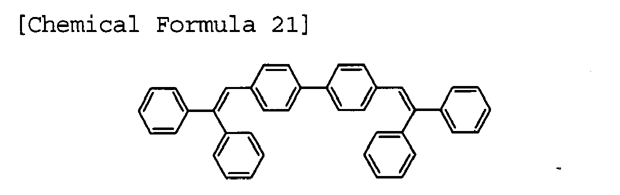

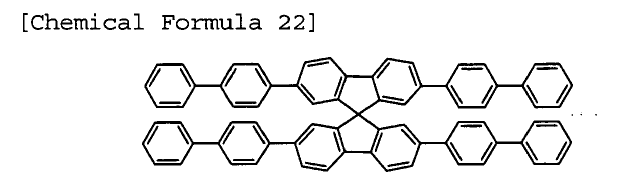

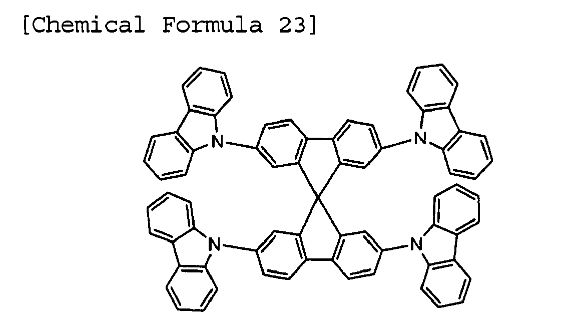

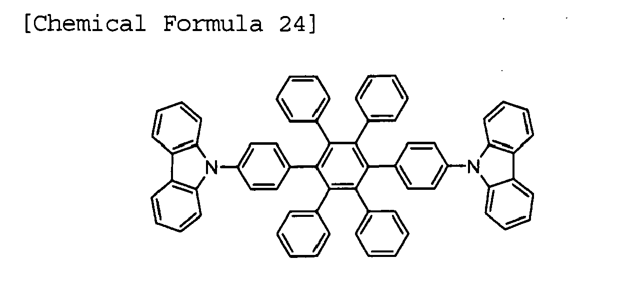

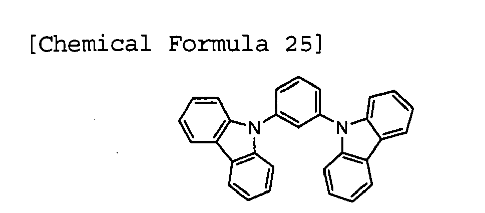

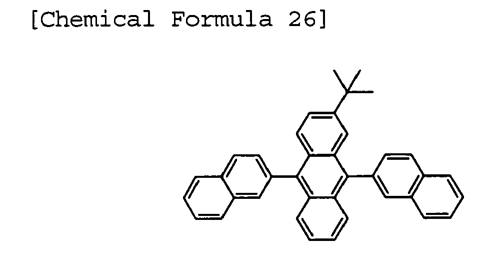

The bipolar material may employ the above-mentioned organic compound which is dispersed in a polymer, an oligomer, or a dendrimer, or which is polymerized, oligomerized or dendrimerized.

Further, it is also possible to use the so-called π-conjugated polymers, such as polyparaphenylene vinylene, polyfluorene, those derivatives, etc., and disconjugate polymers etc. represented by polyvinylcarbazole. Furthermore, so-called conjugated oligomers etc., such as a fluorene oligomer and its derivative, etc. can also be used.

Furthermore, copolymers, such as poly(vinyltriarylaminevinyloxadiazole) etc., where monomers having a hole transport function and an electron transport function exist in the same molecule, and a dendrimer can also be used.

Still further, it is possible to use the above-mentioned bipolar material which is reacted with the oxidizing dopant or reducing dopant as described above to form the hole injection layer or the electron injection layer. Especially, it is preferable that the oxidizing dopant is molybdenum oxide or vanadium oxide.

Although the organic EL material expressed by the above-mentioned general formula (1) can be used for the light emitting layer independently, it may be dispersed together with other hole transport material, light emitting material, electron transport material, etc., or doped, and can also be used combining with any of the above-mentioned organic layers.

In the organic EL element in accordance with the present invention, it is especially preferable that the above-mentioned blue luminescent material is used as a guest material to form the light emitting layer where it is included together with other host material. It is preferable that the concentration of the organic EL material expressed by the above-mentioned general formula (1) in this case is 0.1 - 99 % by weight. Further, it is possible to use it by combining with two or more types of other host materials.

In the case where the organic EL material expressed by the above-mentioned general formula (1) is used as the host material of the light emitting layer, the guest material of the light emitting layer may be a fluorescence or phosphorescence emitting material.

As examples thereof, there may be mentioned multi-ring compounds and those derivatives, such as paraterphenyl, quaterphenyl, etc., styryl compounds and those derivatives, such as [Chemical Formula 15], [Chemical Formula 16], [Chemical Formula 17], etc., a tetraphenylbutadiene derivative, a pyrazoline derivative, an oxadiazole derivative, a coumarin derivative, a styrylamine derivative [Chemical Formula 13], fused multi-ring aromatic hydrocarbon compounds and those derivatives, such as anthracene [Chemical Formula 27], triphenylene, perylene, naphthalene [Chemical Formula 28], pyrene, coronene, chrysene, naphthacene, tetracene, phenanthrene, etc.

Further, it is also possible to use metal chelate complex materials, for example, a quinolinol aluminum complex, a benzoxazole zinc complex, a benzothiazole zinc complex, an azomethine zinc complex, a europium complex, a terbium complex, an iridium complex, a platinum complex, etc., in which Al, Zn, Be, Ir, Pt, Tb, Eu, etc. are provided as central metals, and oxadiazole, thiadiazole, phenylpyridine, and quinoline structures are provided as ligands. In particular, the metal chelate complexes represented by FIrpic [Chemical Formula 29) and those derivatives are mentioned.

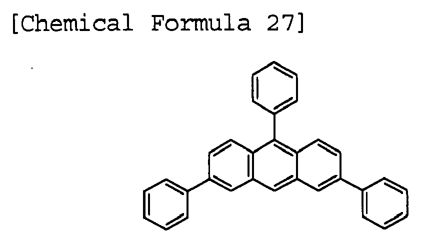

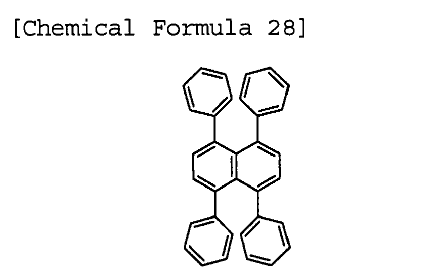

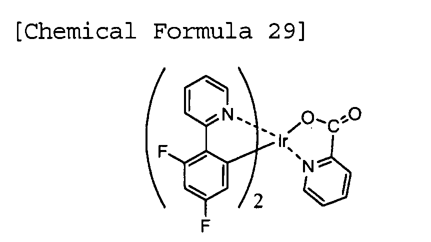

In addition, the light emitting layer may be made of the bipolar material as described above. The organic EL material expressed by the above-mentioned general formula (1) is included in the layer formed of the bipolar material independent or as a mixture, so that blue luminescence can also be obtained.

The bipolar material used for this light emitting layer may be a material which emits fluorescence or phosphorescence by itself. The bipolar material may be contained in any of the hole injection transport layer, the light emitting layer, and the electron injection transport layer.

Further, the material expressed by the above-mentioned general formula (1) can be used as the guest material, and the fluorescence or phosphorescence emitting material can also be used for the host material.

As examples of the host material in this case, there may be mentioned m-phenylene derivatives [Chemical Formula 18] and [Chemical Formula 19], ketone compounds and those derivatives, such as diarylketone etc., carbazole derivatives, such as bis(N-arylcarbazole) [Chemical Formula 14], bis(N-alkenylcarbazole), and bis(N-alkylcarbazole), etc., and an iridium complex represented by Ir(ppz)₃, [Chemical Formula 30).

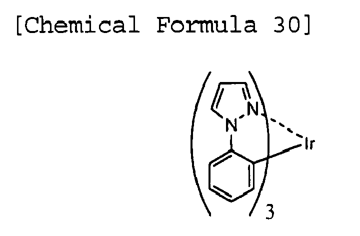

The above-mentioned host material may employ the above-mentioned organic compound which is polymerized, oligomerized or dendrimerized.

Further, it is also possible to use, for example, the so-called π-conjugated polymers, such as polyfluorene etc., and disconjugate polymers etc. represented by polyvinylcarbazole. Furthermore, so-called conjugated oligomers, such as a fluorene oligomer and its derivative, etc. can also be used.

The formation of each of the above-mentioned organic layers can be performed by way of dry processes, such as a vacuum deposition process, a sputtering process, etc., and wet processes, such as an ink-jet process, a casting process, a dip coat process, a bar coat process, a blade coat process, a roll coat process, a photogravure coat process, a flexographic printing process, a spray coat process, etc. Preferably, the film formation is carried out by vacuum deposition.

Further, although a film thickness of each of the above-mentioned layers is suitably determined depending on its conditions in view of adaptability between the respective layers, the whole layer thickness to be required, etc., it is usually preferable to be within a range from 5 nm to 5 micrometers.

In the organic EL element provided with the layer containing the organic EL material expressed by the above-mentioned general formula (1) independently or as a mixture, the blue luminescence having high color purity and provided by the organic EL material expressed by the above-mentioned general formula (1) is combined with green and red luminescence so that white luminescence with high color-rendering properties may be obtained.

As a method of obtaining white luminescence with high color-rendering properties in particular, there may be mentioned a method of adding green luminescence and yellow to orange luminescence to the blue luminescence by the organic EL material expressed by the above-mentioned general formula (1) as complementary colors, a method of causing each of the blue, green, and red luminescent materials containing the material expressed by the above-mentioned general formula (1) to emit light independently, etc.

The above-mentioned green luminescent material or the red luminescent material may be a fluorescence or phosphorescence emitting material.

As examples thereof, there may be mentioned a quinacridone derivative, a squarylium derivative, a porphyrin derivative, a coumarin derivative, a dicyanopyrane derivative, arylamine compounds and those derivatives, such as anthracenediamine etc., fused multi-ring aromatic hydrocarbon compounds and those derivatives, such as perylene, rubrene, tetracene, decacyclene, etc., phenoxazone, a quinoxaline derivative, a carbazole derivative, and a fluorene derivative.

Further, it is also possible to use metal chelate complex materials, for example, a quinolinol aluminum complex, a benzoxazole zinc complex, a benzothiazole zinc complex, an azomethine zinc complex, a europium complex, a terbium complex, an iridium complex, a platinum complex, etc., in which Al, Zn, Be, Ir, Pt, Tb, Eu, etc. are provided as central metals, and oxadiazole, thiadiazole, phenylpyridine, and quinoline structures are provided as ligands. In particular, the metal chelate complexes represented by Ir(ppy)₃ [Chemical Formula 31], Irpiq₃ [Chemical Formula 32] and those derivatives are mentioned.

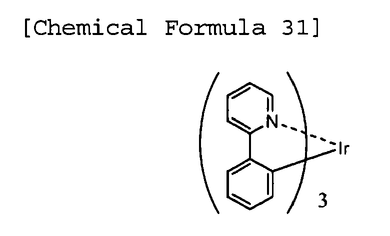

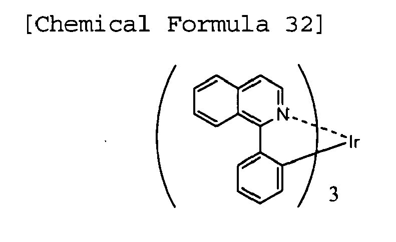

### [Examples]

Hereafter, the present invention will be described still more particularly with reference to Examples. However, the present invention is not limited to the following Examples.

### [Example 1]

### (Synthesis of TMN1357)

TMN1357 was synthesized according to a synthetic scheme as shown below.

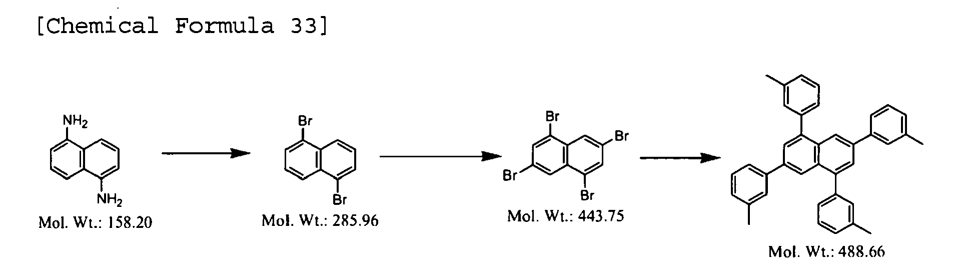

First, 2.0 g (12. 6 mmol) of 1, 5-diaminonaphthalene was suspended in sulfuric acid aqueous solution and 2.0 g (29.0 mmol) of sodium nitrite in an aqueous solution was added under ice-cold conditions, which were diazotized (tetra-azotized). Then, urea in an aqueous solution was added to react with excessive sodium nitrite. This solution was divided into several portions, which were separately added to 5.0 g (34.9 mmol) of CuBr in a hydrobromic acid solution to carry out bromination by way of the Sandmeyer reaction under ice-cold conditions.

It was further reacted at room temperature for 2 to 3 hours, and the precipitated solid was filtered and washed with water and alcohol, and then subjected to vacuum drying by heating.

The resultant black solid was refined by means of a silica gel column using a mixed solvent of n-hexane and chloroform as a developing solvent.

As a result of analysis by MS, ¹H-NMR, the thus obtained product was identified as 1,5-dibromonaphthalene, which was the target. Its yield was 1.32 g (36.6 % yield).

After 1.32g (462 mmol) of 1,5-dibromonaphthalene obtained as described above, 0.30 g (5.37 mmol) of iron powder, and a carbon tetrachloride were placed in a reaction container, 1.85 g (0.60ml, 11.6 mmol) of bromine in a carbon tetrachloride solution was added and stirred to carry out reaction at 55°C for 2 hours.

A sodium thiosulfate solution was supplied to this reaction liquid, unreacted bromine was removed, and extraction was carried out by chloroform. After the extraction, separation was performed, a chloroform layer was washed twice with water. Collecting chloroform, F a precipitated crystal was washed with alcohol.

The thus obtained crystal was identified as a bromination derivative, which was the target, as a result of analysis by ¹H and ¹³C-NMR. Its yield was 1.30 g (63.4 % yield).

1.2 g (2.70 mmol) of 1,3,5,7-tetrabromonaphthalene obtained as described above, 2.21 g (16.2 mmol) of 3-methylphenylboronic acid, 0.78 g (0.675 mmol) of Pd (PPh₃)₄, and a mixed solvent of THF and toluene (1:1) were prepared, to which 2.86g (27.0 mmol) of 2M Na₂CO₃ aqueous solution was added further, and which were heated and refluxed for 48 hours in a nitrogen atmosphere to carry out a coupling reaction.

This reaction solution was poured into water, and extraction was carried out with toluene. A toluene layer was washed twice with water. After collecting the toluene, crude material refined by means of a silica gel column using a mixed solvent of chloroform and n-hexane as a developing solvent.

The thus obtained crystal was identified as TMN1357, which was the target, as a result of analysis by ¹H-NMR. Its yield was 0.91 g (69.0 % yield).

Hereafter, TMN1357 obtained by further subliming and refining the resultant material at 210°C and 3.0×10⁻⁴ Pa was used.

TMN1357 synthesized as described above was formed having a film thickness of 300 nm on a quartz glass substrate by vapor-deposition, and fluorescence analysis was carried out.

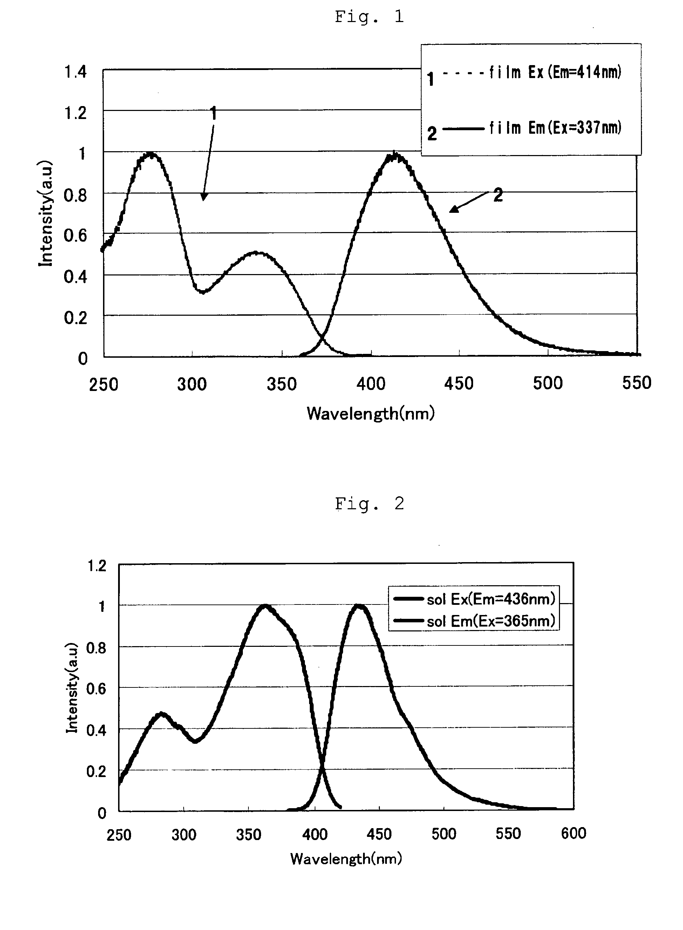

This fluorescence spectrum is shown in Fig. 1.

### [Comparative Example 1]

A blue luminescent material for emitting short wavelength blue light (N20) as shown in the above-mentioned [Chemical Formula 13] was dissolved in chloroform for fluorescence analysis, and fluorescence analysis was carried out with a solution having a concentration of 10⁻⁵ mo/l..

This fluorescence spectrum is shown in Fig. 2.

As a result of the above-mentioned fluorescence analysis, as can be seen from Figs. 1 and 2, TMN1357 in the form of a solid showed the maximum fluorescence wave length on a short wavelength side compared with a dilute solution of N20.

Therefore it was considered that TMN1357 is useful as a host for a blue luminescent material with high color purity as typified by N20.

### [Example 2]

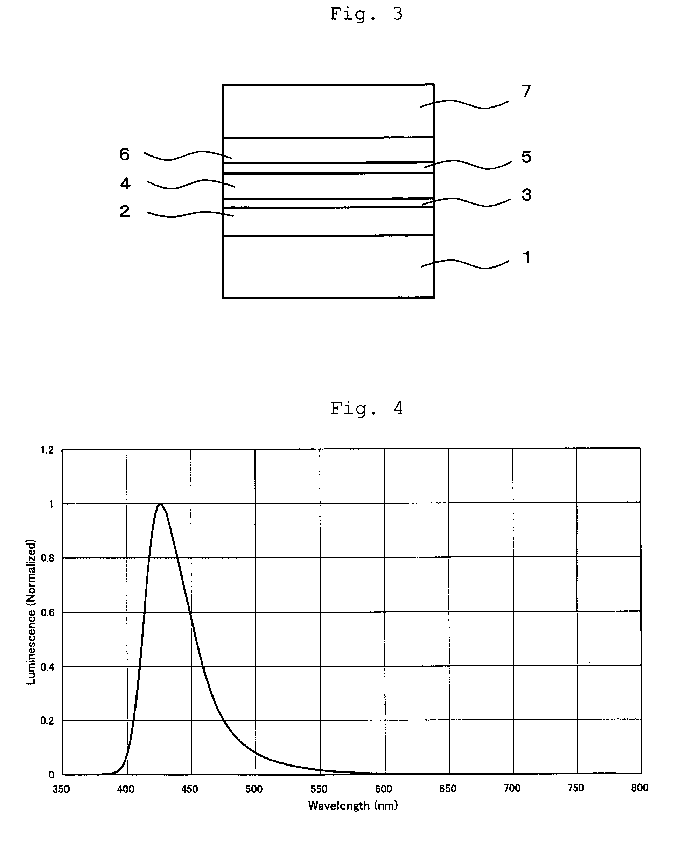

TMN1357 was employed as a host material, and an organic EL element having a light emitting layer in which N20 was doped, using a compound (DTVPF) as shown in the above-mentioned [Chemical Formula 17] as an organic EL material, and having a layer structure as shown in Fig. 3 was prepared according to the following methods.

### (First Electrode)

First, a glass substrate having formed thereon a patterned transparent electroconductive film (ITO) with a film thickness of 150 nm was subjected to washing treatments in the order of ultrasonic cleaning by pure water and a surfactant, washing with running pure water, ultrasonic cleaning by a 1:1 mixed solution of pure water and isopropyl alcohol, and boiling washing by isopropyl alcohol. This substrate was slowly pulled up from the boiling isopropyl alcohol, and dried in isopropyl alcohol vapor, and, finally ultraviolet ozone cleaning was performed.

This substrate was used as an anode 1 and placed in a vacuum chamber which was evacuated to 1×10⁻⁶ Torr. In this vacuum chamber, each molybdenum boat filled up with a vapor deposition material and a vapor deposition mask for forming a film in a predetermined pattern were placed, the above-mentioned boat was electrically heated, and the vapor deposition material was evaporated to thereby form each organic layer one by one.

### (Hole Injection Transport Layer)

By using the compound (DTVPF) shown in the above-mentioned [Chemical Formula 19] as an hole transport material together with molybdenum trioxide (MoO₃), the respective boats were electrically heated at the same time to carry out co-deposition. A hole injection layer 2 of DTVPF: MoO₃=67:33 was formed to have a film thickness of 10 nm.

Next, a hole transport layer 3 made only of DTVPF was formed to have a film thickness 56 nm.

### (Light Emitting Layer)

A light emitting layer 4 of TMN1357:N20=94:6 was formed to have a film thickness of 15 nm.

### (Electron Injection Transport Layer)

An electron transport layer 5 made of DTVPF was formed to have a film thickness of 38 nm, on which an electron injection layer 6 of DTVPF:Liq=50:50 as an electron transport material was formed to have a film thickness of 10 nm.

### (Second Electrode)

While maintaining the vacuum chamber at a vacuum, masks were replaced to install masks for cathode vapor deposition. An aluminum (Al) layer was formed having a film thickness of 100 nm to be a cathode 7.

The vacuum chamber was returned to ambient pressure, and the substrate on which each layer was deposited as described above was taken out, and it was moved in a glove box in which nitrogen substitution was carried out. By using a UV curing resin, it was sealed with another glass board to obtain an organic EL element.

A layer structure of this element may be simplified and shown as being ITO (150 nm) / DTVPF: MoO₃ (10 nm, 67:33) / DTVPF (56 nm) / TMN1357:N20 (15 nm, 94:6) / DTVPF (38 nm) / DTVPF:Liq (10 nm, 50:50) / Al (100 nm).

A light emission spectrum of this organic EL element is shown in Fig. 4 when this organic EL element was supplied with a direct current of 100 A/m²_{.}

As shown in Fig. 4, pure blue luminescence due to N20 was obtained.

Further, the chromaticity of this luminescence color was (x, y) = (0.157, 0.044) in CIE coordinates (100 A/m²), and it was confirmed as being blue luminescence with high color purity.

### [Comparative Example 2]

The compound (TBADN) as shown in the above-mentioned [Chemical Formula 26] was employed as a host material, and an organic EL element having a light emitting layer in which N20 was doped was prepared similarly to Example 2.

A layer structure of this element may be simplified and shown as being ITO (150 nm) / NS21: MoO₃ (59 nm, 90:10) / NS21 (10 nm) / TBADN:N20 (30 nm, 97:3) / BAlq(5 nm) /DPB(16nm) /DPB:Liq (5 nm, 74:26) / Al (100nm).

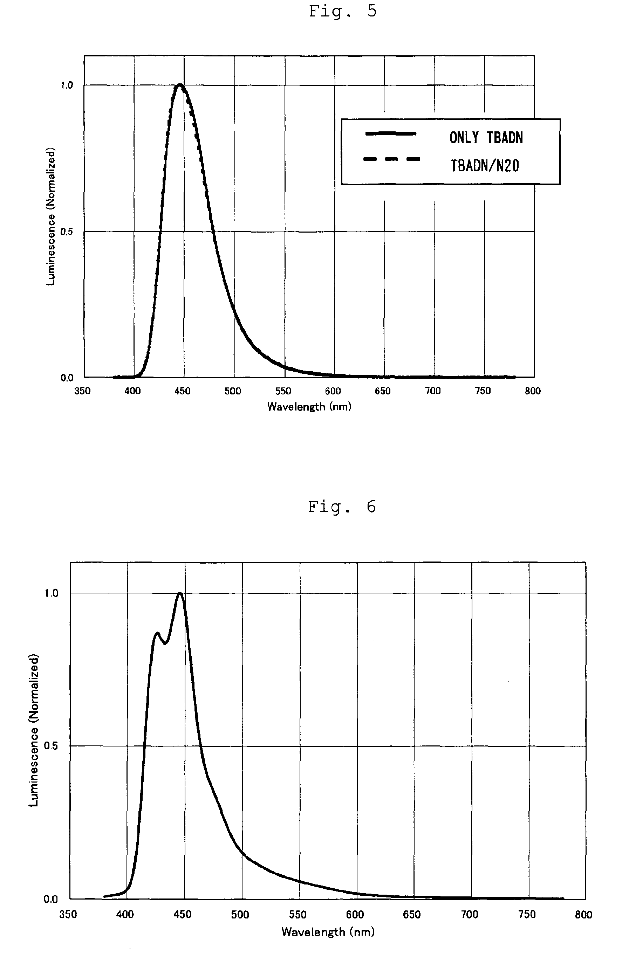

A light emission spectrum of this organic EL element is shown in Fig. 5 when this organic EL element was supplied with a direct current of 100 A/m². For comparison, the case where the N20 was not doped (only TBADN) is also shown in Fig. 5

As can be seen from Fig. 5, in the case where TBADN was used as the host material, blue luminescence of the N20 origin was not obtained.

Further, the chromaticity of this luminescence color was (x, y) = (0.151, 0.080) in CIE coordinates (100 A/m²), and blue luminescence had low color purity.

### [Example 3]

TMN1357 was employed as a host material, and an organic EL element having a light emitting layer in which a blue luminescent material for emitting short wavelength blue light (TPA)as shown in the above-mentioned [Chemical Formula 27] was doped was prepared similarly to Example 2.

A layer structure of this element may be simplified and shown as being ITO (150 nm) / DTVPF: MoO₃ (10 nm, 67:33) / DTVPF (56 nm) / TMN1357:TPA (20 nm, 98:2) / DTVPF (15 nm) / DTVPF:Liq (10 nm, 50:50) / Al (100 nm).

A light emission spectrum of this organic EL element is shown in Fig. 6 when this organic EL element was supplied with a direct current of 1000 A/m².

As shown in Fig. 6, pure blue luminescence due to TPA was obtained.

Further, the chromaticity of this luminescence color was (x, y)=(0.165, 0.083) in CIE coordinates (1000 A/m²), and it was confirmed as being blue luminescence with high color purity.

### [Comparative Example 3]

TBADN was employed as a host material, and an organic EL element having a light emitting layer in which TPA was doped was prepared similarly to Example 2.

A layer structure of this element may be simplified and shown as being ITO (150 nm) / DTVPF: MoO₃ (10 nm, 67:33) / DTVPF (56 nm) / TBADN:TPA (20 nm, 98:2) / DTVPF (15 nm) / DTVPF:Liq (10 nm, 50:50) / Al (100 nm).

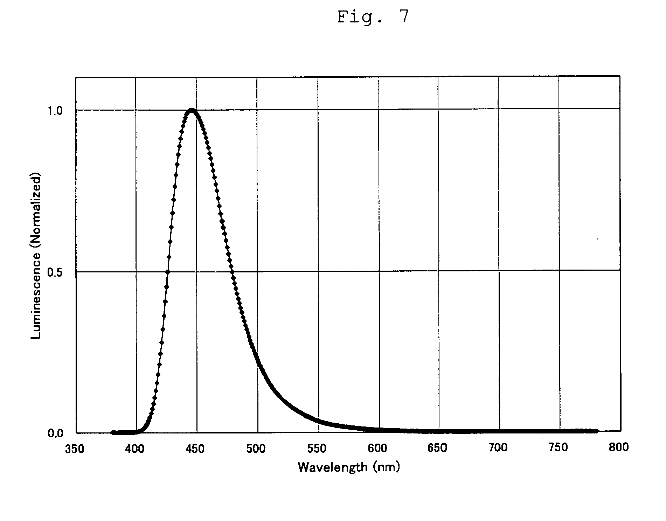

A light emission spectrum of this organic EL element is shown in Fig. 7 when this organic EL element was supplied with a direct current of 1000 A/m².

As shown in Fig. 7, pure blue luminescence due to TBA was not obtained.

Therefore, it was confirmed that TMN1357 was excellent as the host for a blue luminescent material compared with TBADN according to above example 3 and comparative example 3.

As described above, it is confirmed that the organic EL material expressed by the general formula (1) in accordance with the present invention provides the blue luminescence excellent in color purity, is highly practical, and is expected to be applied to a light source and a display apparatus which require high color purity.

## Claims

1. An organic electroluminescence material expressed by following general formula (1) . 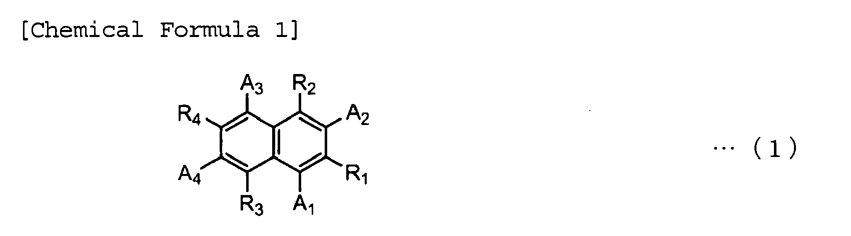 (where R₁-R₄ are selected from the group consisting of hydrogen, an alkyl group, a cycloalkyl group, an alkoxy group, a cycloalkoxy group, and an aryloxy group, and they may be the same groups or the groups different from one another, and A₁-A₄ are selected from the group consisting of a phenyl group which is either substituted or unsubstituted and a 5 or 6 member heterocyclic ring group which is either substituted or unsubstituted, and they may be the same groups or the groups different from one another.)

2. An organic electroluminescence element having one or a plurality of organic layers including a light emitting layer between a pair of electrodes, wherein at least one layer of said organic layers contains the organic electroluminescence material as claimed in claim 1 independently or as a mixture.

3. The organic electroluminescence element as claimed in claim 2, wherein at least one layer of said organic layers is a light emitting layer containing the organic electroluminescence material as a host material as claimed in claim 1 and a fluorescence or phosphorescence emitting material as a guest material.

4. The organic electroluminescence element as claimed in claim 2, wherein said electrode is such that a transparent electroconductive thin-film is formed on a transparent substrate.
